# EUROPEAN PATENT SPECIFICATION

(11) **EP 1 806 130 B1**
(45) Date of publication and mention of the grant of the patent: **31.03.2010**
(21) Application number: 06000329.0
(22) Date of filing: 09.01.2006
(51) Int. Cl.: A61K 9/20, A61K 9/16, A61K 31/415, A61P 9/12

(54) **Solid pharmaceutical composition comprising irbesartan**
Irbesartan enthaltende feste Zubereitung
Préparations solides comprenant du irbesartan

(43) Date of publication of application: 11.07.2007
(73) Proprietor: KRKA, D.D., Novo Mesto, 8501 Novo mesto (SI)
(72) Inventor: Vrbinc, Miha, 8000 Nova mesto (SI); Jakse, Renata, 8220 Smarjeske Toplice (SI); Bevec, Franci, 8310 Sentjernej (SI); Zupancic, Silvo, 8000 Novo mesto (SI)
(74) Representative: HOFFMANN EITLE

(56) References cited:
- EP-A- 1 275 391
- WO-A-2005/025566
- US-A1- 2005 019 398
- US-A1- 2005 271 720

## Description

### Field of the Invention

The invention relates to a solid pharmaceutical composition, which comprises as an active ingredient irbesartan hydrochloride, alone or in combination with a diuretic, i.e. hydrochlorothiazide, and a process for its preparation. In particular, it relates to a composition and a process wherein the active ingredient retains its structure and stability. In a preferred aspect, the composition is surfactant-free.

### Background of the Invention

2-Butyl-3-[[2'-(1H-tetrazol-5-yl)[1,1'-biphenyl]-4-yl] methyl] -1, 3-diazaspiro [4,4] -non:::J.-:-:en-4 -one (irbesartan) is a non-peptide tetrazole derivative (empirical formula C₂₅H₂₈N₆O, molecular weight 428.5 g/mol) of the formula shown below and has angiotensin II type 1 receptor (AT₁) antagonistic activity:

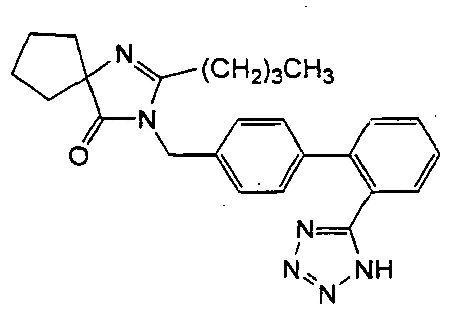

Processes for the preparation of irbesartan are described in e.g. EP 0 454 511, WO 2004/007482, EP 0 708 103, EP 1 089 994, SI P-200500004, SI P-200500132 and WO 03/050110.

The preparation of irbesartan hydrochloride (in the following irbesartan-HCl) from e.g. free irbesartan can be easily achieved by the skilled person, and an example of a suitable process is described later herein. Also, suitable processes are described in the patent applications SI P-200400220 and SI P-200400292.

EP 0 454 511 A1 discloses a broad variety of N-substituted heterocyclic compounds including irbesartan, and solid pharmaceutical composition in form of tablets comprising these compounds. These tablets also comprise a vehicle such as gelatin, starch, lactose, magnesium stearate, talc and gum arabic, and may be coated with sucrose, a cellulose derivative or other appropriate substances.

EP 0 747 050 A1 describes irbesartan as a fluffy material having relatively low bulk and tap densities, making it difficult to formulate a substantial amount of the drug (e.g. 75-300 mg) into a small tablet with impairing uniformity of weight, hardness and other desirable tablet properties. The definition of irbesartan given in this reference includes pharmaceutically acceptable salts thereof without defining them in detail. Preferred compositions comprise, among others, 20-70 wt.-% of irbesartan and 0.2-6 wt.-% of surfactant. The method for the preparation of the tablets described in EP 0 747 050 A1 is aqueous granulation.

WO 05/025566 is directed to irbesartan-containing oral pharmaceutical formulations having improved dissolution profiles, and especially dissolution of 80% or more 15 minutes after dosing and of 90% or more 30 minutes after dosing. To achieve this goal the formulations are prepared by wet granulation of compositions comprising, among others, irbesartan (which is described as a white crystalline powder that is slightly soluble in alcohol and CH₂Cl₂ and insoluble in water), alcohol as wetting agent, and other ingredients including a surfactant, such as poloxamer 188^{®}.

US 2005/0271720 A1 discloses pharmaceutical compositions comprising a high relative amount of irbesartan (>70 wt.-%) and at least one excipient, such as a binder, surfactant, diluent, disintegrant, anti-adherent or lubricant. In a preferred embodiment poloxamer 188^{®} (a surfactant) is present in an amount of 1.06-2.0 wt.-%, and also the concrete examples include this component.

WO 97/17064 describes a pharmaceutically acceptable freeze-dried formulation consisting of a crystalline phase and an amorphous phase and including at least one non-protein drug, which further contains mannitol and alanine in a weight ratio of 0.1:1 to 1:1. Among a large variety of suitable non-protein drugs irbesartan is mentioned as an example of the class of angiotensin II antagonists.

CN 1 415 298 relates to irbesartan capsules consisting of irbesartan, hydrochlorothiazide, pre-gelatinized corn starch, lactose, microcrystalline cellulose, crosslinked carboxymethylcellulose sodium, SiO₂, magnesium stearate and polyvinyl pyrrolidone.

US 2005/0019398 A1 concerns granules for the production of flash-melt pharmaceutical oral dosage forms for a broad variety of active agents. The prepared dosage forms, especially tablets, disintegrate in the mouth in under about twenty five seconds.

In general, in the past the specific physical and chemical properties of irbesartan, especially its low solubility in aqueous media, gave rise to problems in the formulation thereof into formulations suitable for an effective oral administration. Hence, a need exists for formulations of irbesartan for effective oral delivery, which provide for an appropriate disintegration time and/or an appropriate solubility and/or dissolution profile of the active principle.

In addition, it is desired that these effects can be achieved with a formulation that has an improved processability, and/or involves excipients, which are easily available at low cost, and/or economically preferable technical processes for its preparation.

### Summary of the Invention

Accordingly, the present invention provides a solid pharmaceutical formulation comprising, as an active ingredient, irbesartan hydrochloride, mannitol, low-substituted hydroxypropylcellulose and at least one lubricant selected from macrogol, talc and hydrogenated castor oil.

In solid dosage forms, surfactants have been widely shown to enhance drug dissolution rate. This may be due to wetting effects, resulting in increased surface area, effects on solubility and effective diffusion coefficient, or a combination of these effects.

This and other advantages (e.g. improvement of solubility, thermodynamic activity, diffusion and disintegration of surfactants) and their influence on rate and extent of drug absorption were generally known. According to a preferred aspect of the present invention, however, it has been surprisingly found that the use of a surfactant may be omitted if a disintegrant is present.

Hence, in a preferred aspect the present invention aims at a solution to the problems described above by providing pharmaceutical formulations, which do not contain a surfactant.

According to a further preferred aspect, the formulations of the invention comprise, as an active ingredient, irbesartan hydrochloride having an average particle size of the active ingredient less than 250 µm, and the maximum diameter is 2000 µm.

The active ingredient is irbesartan-HCl. Optionally, a diuretic, i.e. hydrochlorothiazide (HCTZ) is also included in the formulation.

### Brief Description of the Drawings

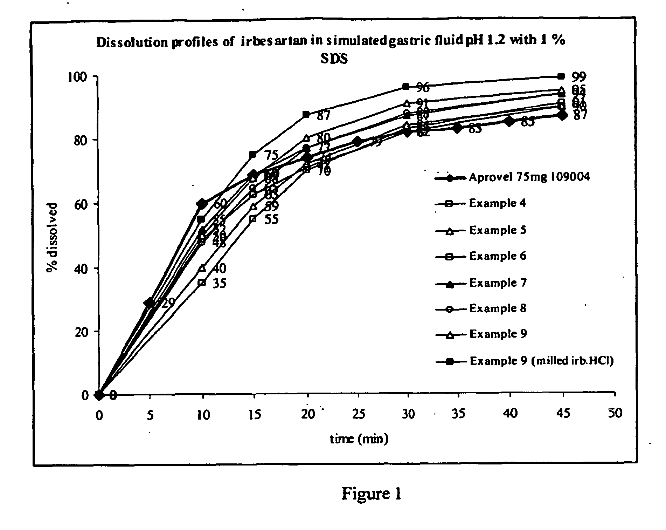

Figure 1 depicts the comparison of irbesartan dissolution profiles of Aprovel 75 mg tablets (a pharmaceutical dosage form currently on the market) and Examples 4-9 (tablet cores (4c)-(9c)) of the present invention.

### Detailed Description of the Invention

In the following description, any reference to irbesartan is intended to include irbesartan-HCl, if not explicitly indicated to the contrary.

The solid pharmaceutical formulations of the invention comprise irbesartan hydrochloride as an active ingredient, mannitol, low-substituted hydroxypropylcellulose and at least one lubricant selected from macrogol, talc and hydrogenated castor oil.

The irbesartan hydrochloride may be present in a crystalline form or in a non-crystalline form.

Crystalline forms include anhydrous or hydrated forms, preferably sesquihydrates, such as irbesartan-HCl sesquihydrate. Non-limiting examples of methods for the preparation of crystalline irbesartan are disclosed in EP 0 454 511 B1, WO 2004/007482, EP 0 708 103 or EP 1 089 994.

Non-crystalline forms include e.g. amorphous irbesartan, which can be prepared according to any suitable known process, e.g. by evaporation, milling, heating, lyophilization, spray-drying etc., and a specific example is the process described in WO 03/050110.

Particularly preferred do formulations comprise irbesartan-HCl sesquihydrate, since such formulations have been found to be very stable.

In the present description and examples the crystal water content of irbesartan, as determined by the Karl-Fischer method, performed according to Ph.Eur., is included in the total amounts disclosed, where appropriate.

For irbesartan-HCl as the active ingredient the dissolution profile and disintegration is surprisingly further improved, and the solubility of irbesartan-HCl is higher in several dissolution media such as 0.01 M HCl or simulated gastric juice (pH = 1.2), as compared to the free irbesartan.

A preferred formulation according to the present invention is a surfactant-free solid pharmaceutical formulation. In the context of the present invention, a "surfactant" is a substance, which, at low concentrations, adsorbs onto the surfaces or interfaces of a system and alter the surface or interfacial free energy and the surface or interfacial tension, and which is amphipathic in nature, i.e. it possess both polar (hydrophilic) and nonpolar (hydrophobic) regions in the same molecule.

Especially, surfactants not to be present in the preferred formulations of the present invention are the following, which can be classified into four groups, based on the nature of the hydrophilic group within the molecule.
1. Anionic surfactants, where the hydrophilic group carries a negative charge, such as carbonyl (RCOO⁻), sulfonate (RSO₃⁻) or sulfate (ROSO₃⁻) . Examples of pharmaceutical importance include potassium laurate, and sodium lauryl sulfate.
2. Cationic surfactants, where the hydrophilic group carries a positive charge (e.g., quaternary ammonium halides, R₄N⁺Cl⁻). Examples of pharmaceutical importance include cetrimide, a mixture consisting mainly of tetradecyl (ca. 68%), dodecyl (ca. 22%), and hexadecyltrimethylammonium bromides (ca. 7%), as well as benzalkonium chloride, a mixture of alkylbenzyldimethylammonium chlorides of the general formula [C₆H₅CH₂N⁺(CH₃)₂R]Cl⁻, where R represents a mixture of the alkyls from C₈H1₇ to C₁₈H₃₇.
3. Ampholytic surfactants (also called zwitterionic surfactants), where the molecule contains, or can potentially contain, both a negative and a positive charge, such as sulfobetaines (RN⁺(CH₃)₂CH₂CH₂SO₃⁻). Examples of pharmaceutical importance include N-Dodecyl-N,N-Dimethylbetaine, C₁₂H₂₅N⁺(CH₃)₂CH₂COO⁻.
4. Nonionic surfactants, where the hydrophilic region carries no charge but derives its water solubility from highly polar groups such as hydroxyl or polyoxyethylene (OCH₂CH₂O-) groups. Examples of pharmaceutical importance include polyoxyethylated glycol monoethers (e.g. cetomacrogol), sorbitan esters (Spans^{®}), polysorbates (Tweens^{®}) and poloxamers (nonionic polyoxyethylene-polyoxypropylene copolymers) having chemically similar compositions and differing only in the relative amounts of units derived from propylene oxide and ethylene oxide.

Lubricants, glidants and anti-adherents as used in solid pharmaceutical compositions of the present invention, are herein not defined as surface-active agents (surfactants).

The present formulations comprising irbesartan hydrochloride may optionally further comprise a diuretic, i.e. hydrochlorothiazide (HCTZ). This can be included either into the intragranular phase of a granulate and/or into the extragranular phase, i.e. as an additive to a granulate. The granulate may be, for example, a granulate for tablet compression or a granulate for filling capsules. Further, the diuretic can be included in coating layer provided on a core. The core may be individual granules of a granulate, or a core obtained by compressing a granulate or powdery material. These modes of inclusion can be used in combination with each other.

The formulations of the invention can be in the form of tablets, pills, powders, lozenges, sachets, soft and hard gelatin capsules, suppositories etc. The dosage form is preferably suitable for oral application.

The formulations of the invention are preferably provided in a unit dosage form, each dosage containing about 1-300 mg, more preferably about 50-200 mg, and even more preferred about 75-150 mg (especially about 100 mg) of irbesartan in its free form (e.g. a therapeutic dose of 100 mg free irbesartan corresponds to 114.7 mg of irbesartan-HCl*1.5 H₂O). The term »unit dosage form« refers to physically discrete units suitable as unitary dosages for humans and other mammals, each unit containing a predetermined quantity of irbesartan calculated to produce the desired therapeutic effect, in association with a suitable pharmaceutical excipient.

In yet another preferred embodiment of the present formulations the average particle size of the irbesartan hydrochloride is less than 250 µm, more preferably less than 200 µm, most preferably less than 150 µm. The maximum particle diameter is preferably 2000 µm or less, more preferably 1000 µm or less.

The term "average particle size" as used herein refers to the volume mean particle diameter, and "maximum diameter" refers to the maximum volume particle diameter, both determined by laser light scattering of a sample comprising 100-800 mg of substance dispersed in 5-8 ml of vegetable oil (i.e. sunflower oil) and not containing any solubilizers or surfactants, using a Malvern Mastersizer instrument MS2000.

### Excipients

The pharmaceutical excipients used in and for the preparation of the present pharmaceutical formulations particularly include diluents, binders, disintegrants and lubricants. Other and further excipients can also be contained.

Examples of diluents as excipients include microcrystalline cellulose, powdered cellulose, lactose (anhydrous and monohydrate), compressible sugar, fructose, dextrates, sugar alcohols such as mannitol, sorbitol, maltitol, xylitol, lactitol, or other sugars such as saccharose, raffinose, trehalose, fructose or mixture thereof, siliconized microcrystalline cellulose, calcium hydrogen phosphate, calcium carbonate, calcium lactate and any mixtures thereof. Preferred excipients are microcrystalline cellulose, lactose and mannitol, and any mixtures thereof.

Examples of binders as excipients include polyvinylpyrrolidone, microcrystalline cellulose, hydroxyethylcellulose, hydroxypropylcellulose, hydroxypropylmethylcellulose or other cellulose ethers, starch, pre-gelatinized starch, polymethacrylate, and any mixtures thereof. Preferably, the binder has good water solubility, and especially, there is a strong preference for binders with a very good solubility in cold water, such as povidone of different K-values and hydroxypropylcellulose, i.e. partially substituted poly(hydroxypropyl)ether of cellulose and/or low substituted poly(hydroxypropyl)ether of cellulose.

Examples of disintegrants as excipients include pre-gelatinized starch, sodium starch glycollate, microcrystalline cellulose, carboxymethylcellulose sodium (CMC-Na) or calcium (CMC-Ca), cross-linked CMC-Na, polacrilin potassium, low-substituted hydroxypropylcellulose and any mixtures thereof. Preferably, the disintegrant comprises cross-linked CMC-Na and/or low-substituted hydroxypropylcellulose.

Examples of lubricants as excipients include stearic acid or stearic acid salts, such as magnesium stearate, magnesium palmitate, magnesium oleate, hydrogenated vegetable oil, hydrogenated castor oil, talc, sodium stearyl fumarate, macrogols, and any mixtures thereof. Preferably, the lubricant comprises at least one component selected from stearic acid, magnesium stearate, hydrogenated vegetable oil, hydrogenated castor oil, macrogols and talc.

### Composition

If not indicated otherwise, all percentages given herein are wt.-%, based on the total weight of the formulation.

Preferably, the present formulations comprise irbesartan hydrochloride in an amount of 20-77 %, more preferably 25-75 %, even more preferably 30-70 %; still more preferably 30-65 %, and most preferably 30-60 %.

According to a more preferred embodiment the pharmaceutical formulations of the invention comprise:
- irbesartan hydrochloride:: 20-77 %, more preferably 25-75 %, even more preferably 30-70 %; still more preferably 30-65 %, and most preferably 30-60 %.
- diluent: 20-77 %, more preferably 25-75 %, most preferably 30-70 %;
- binder:: 1-20 %, more preferably 2-16 %, most preferably 3-12 %,
- disintegrant:: 1-30 %, more preferably 2-25 %, most preferably 3-20 %,
- lubricant:: 1-25 %, more preferably 2-20 %, most preferably 3-15 %, and
- diuretic:: 0-20%, more preferably 2-10%, most preferably 2-8%.

Preferably, the irbesartan in the above formulations is irbesartan-HCl, more preferably irbesartan hydrochloride sesquihydrate. Also, in the above formulations the diluent is mannitol.

Even more preferred are formulations comprising the preferred excipients defined above.

The formulations of the invention are preferably in form of a coated or uncoated tablet, such as a fast disintegrating tablet or orally disintegrating tablet, a capsule or in form of pellets. The composition can also take the form of a powder mixture, a granulate or mini tablets filled in capsules. An immediate release composition is preferable.

Optionally, cores/tablets can be coated with conventional materials used for film coating, i.e. as described in G. Cole (ed.), Pharmaceutical Coating Technology (1995). Film coating formulations usually contain the following components:
i) at least one polymer,
ii) at least one plasticizer,
iii) at least one colorant and/or opacifier, and
iv) at least one vehicle (solvent).

In the film coating suspensions minor quantities of flavors, surfactants and waxes can be used. The majority of the polymers used in film coating are either cellulose derivatives, such as the cellulose ethers, or acrylic polymers and copolymers. Occasionally encountered are high molecular weight polyethylene glycols, polyvinyl pyrrolidone, polyvinyl alcohol and waxy materials. Their function usually is to prevent bad mouthfeel and/or taste and in some cases degradation, e.g. oxidation of the active ingredients and/or excipients used.

Typical cellulose ethers, which may be applied as coatings are hydroxyethylcellulose, hydroxypropylcellulose, hydroxypropylmethylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

The commonly used plasticizers can be categorized into polyols (such as glycerol, propylene glycol and macrogols), organic esters (such as phthalate esters, dibutyl sebacate, citrate esters and triacetin), and oils/glycerides (such as castor oil, acetylated monoglycerides and fractionated coconut oil).

Colorants/opacifiers include organic dyes and their lakes, inorganic colors and natural colors, including water-soluble colors and water-insoluble colors (pigments).

Different materials from each group can also be combined in defined ratios. Film coating suspensions can also be used as ready-to-make preparations, which are commercially available.

Film coating dispersion can be prepared by using different vehicles (solvents, such as water, alcohols, ketones, esters, chlorinated hydrocarbons and mixtures thereof. A preferred vehicle (solvent) is water.

Particularly preferred is a composition of the coating suspension (as) comprising, in terms of wt.-%, based on the dry material, 1-99 % (preferably 1-95 %) polymer, 1-50 % (preferably 1-40 %) plasticizer and 0.1-20 % (preferably 0.1-10 %) colorant/opacifier.

Conventional equipment for applying a coating, such as a Wurster coating system or conventional coating pans can be used.

In a particularly preferred embodiment of the present invention, the solid pharmaceutical formulation comprises irbesartan or irbesartan hydrochloride (most preferably irbesartan hydrochloride sesquihydrate), mannitol, one or two hydroxypropylcelluloses (HPC). The HPC can be unsubstituted but is more preferably substituted. A low degree of substitution (0.5 or less, more preferred 0.2 or less) is preferred. These preferred formulations may also comprise HCTZ as a further active ingredient.

In these preferred formulations, the average particle size of irbesartan or its hydrochloride (sesquihydrate) is preferably less than 250 µm and the amount of active ingredient(s) is preferably between 20 and 60%. Mannitol is used in an amount of 20-77%, at least one lubricant selected from macrogol, talc and hydrogenated castor oil is used in a total amount of 1-25% and one or two HPCs (preferably two types of HPC are used) are present in an amount of 1-20% in total. Each of the above amounts is based on the total weight of the formulation. The formulations may preferably be coated. Formulations according to this preferred embodiment of the present invention provide a particularly good stability and pharmacokinetic profile and have adequate dissolution kinetics.

### The Process of the Invention

The present pharmaceutical formulations are prepared by known technological procedures, e.g. by compression or wet granulation, using well-known and readily available excipients. In the preparation of the formulations comprising irbesartan the active ingredient will usually be mixed or diluted with one or more excipient(s). When the excipient serves as a diluent, it may be a solid or liquid material, which acts as a vehicle or medium for the irbesartan component.

It has been found out that the inventive solid oral dosage form, which is prepared by wet granulation, is stable at ICH stability testing conditions. Further, drying a granulate, prepared in a wet granulation process, in a fluid-bed dryer enables the preparation of round-shaped particles of granulate consisting of irbesartan and excipient(s), which provide a reproducible and processable formulation. This can otherwise represent a problem due to the high irbesartan content in the solid dosage form, e.g. 20-77% in the core.

The wetting of a mixture of irbesartan and excipient(s) can be performed in conventional granulation equipment by contacting water, alcohol, a mixture thereof or of an aqueous, alcoholic or aqueous/alcoholic granulating liquid onto one or more excipient(s) by conventional pharmaceutical techniques, such as spraying or direct addition during a mixing operation in a proper mixing device, e.g. high-shear mixer. Preferred diluents used in the technological process of wet granulation, are lower alcohols, e.g. C₁₋₆-alkanols.

The terms »aqueous, alcoholic or aqueous/alcoholic granulating liquid« refers to a mixture containing water (preferably purified, demineralized or distilled water) and/or lower alcohols (preferably C₁₋₆-alkanols, such as methanol, ethanol or isopropanol) as diluents and a solid substance which is dispersed, suspended or dissolved therein. The solid substance can have known functions of excipients used in the present formulations, such as those of a suspending agent or binding agent, preferably a binding agent.

Mixing of the excipient(s) with the irbesartan may be effected in a conventional device used for mixing of powders, e.g. a motionless (passive) mixer, fluidized bed mixer, diffusion mixer, biconic diffusion mixer, uniconic mixer, biconic mixer, turbular mixer, cubic mixer, planetary mixer, Y-shaped mixer, V-shaped mixer or high-shear mixer.

For drying of granulate, conventional drying devices such as a fluid-bed dryer or drying chambers (under vacuum or at ambient pressure) can be used.

In the process of the invention as described above, the compression, in particular to cores/tablets, can be effected using an automatic rotary compressing machine from different manufacturers of equipment for use in pharmaceutical industry.

Conventional equipment can be used for applying film coating, such as fluid bed (e.g. Wurster coating system) or conventional coating pans for use in pharmaceutical industry.

The process for preparing the pharmaceutical formulations of the invention can be carried out as a granulation process or direct compression process. In each case the irbesartan compound is first prepared according to a suitable synthetic process and then purified, e.g. by crystallization or other means. Then, the size of irbesartan particles is determined and if it is found that there are particles having a diameter of more than 2000 µm (or more than 1000 µm in the case of a more preferred embodiment) the irbesartan is milled or crunched to a smaller size.

A preferred embodiment of the invention is a wet granulation process (A) comprising the following steps:
(i) providing irbesartan hydrochloride having a maximum particle diameter of not more than 2000 µm,
(ii) preparing a compression mixture by using a wetting liquid comprising alcohol, optionally water, a mixture thereof or an aqueous, alcoholic or aqueous/alcoholic granulating liquid by
   - (a1) granulating a mixture of one or more excipient(s) and the wetting liquid to obtain granulate, and (a2) adding the irbesartan hydrochloride and optionally further excipient(s) to the granulate to obtain a compression mixture;
   - (b1) granulating a mixture of one or more excipient(s), the irbesartan hydrochloride and the wetting liquid to obtain granulate, and (b2) optionally adding further excipient(s) to the granulate to obtain a compression mixture; or
   - (c1) granulating a mixture of one or more excipient(s), a portion of the irbesartan hydrochloride and the wetting liquid to obtain granulate, and (c2) adding the remaining irbesartan and optionally further excipient(s) to the granulate to obtain a compression mixture; and
(iii) compressing the compression mixture to the desired form.

In this process the step (ii) includes three alternatives. In the steps (a1)/(a2) all irbesartan hydrochloride is added as extragranular phase after the granulation (a1), in steps (b1)/(b2) all irbesartan is included into the granulate in (b1), and in steps (c1)/(c2) the irbesartan is divided between granulate and extragranular phase.

Also, hydrochlorothiazide can be optionally included in the present irbesartan formulations. Thus, depending on the alternative chosen for step (ii), in the present wet granulation process hydrochlorothiazide can be optionally added to any of the steps (a1) and/or (a2), (b1) and/or (b2), and (c1) and/or (c2). Hence, the hydrochlorothiazide, if added, can be present exclusively within the granulate, it can be added exclusively as extragranular phase after the granulation, or it can divided between granulate and extragranular phase, independently from the distribution of the irbesartan achieved by selecting the desired alternative (a1) / (a2), (b1) / (b2) or (c1) / (c2) of step (ii).

Another preferred embodiment of the process of the invention is a direct compression process (B) comprising the steps of:
(i) providing irbesartan hydrochloride having a maximum particle diameter of not more than 2000 µm,
(ii') mixing the irbesartan hydrochloride and one or more excipient(s) to give compression mixture, and
(iii) compressing the compression mixture to the desired form.

Again, hydrochlorothiazide can be optionally included. If so, it is added as further component into the mixing step (ii').

To the compressed product obtained in step (iii) of either the wet granulation process (A) or the direct compression process (B) a coating may be optionally applied in a subsequent step (iv).

Further, the excipients present in the formulations of the invention, such as binders, disintegrants, lubricants or diluents, can be divided between the intragranular phase, the extragranular phase and optionally the coating layer in the wet granulation process (A), and between the intragranular phase and optionally the coating layer in the direct compression process (B).

According to a preferred embodiment, the amount of irbesartan hydrochloride in the present formulations is 75 mg, 150 mg or 300 mg per dosing unit (in terms of free irbesartan hydrochoride). The optional additional amount of hydrochlorothiazide in these formulations is preferably 12.5 mg or 25 mg per dosing unit. Any combinations of these amounts are possible, i.e. the ratio of irbesartan-HCl to hydrochlorothiazide (mg:mg) may preferably be 75:12.5, 150:12.5, 300:12.5, 75:25, 150:25 or 300:25.

### EXAMPLES

The following examples illustrate the invention and are not intended to restrict the scope of the invention in any way.

The drying loss determined in the examples was measured using a Mettler Toledo HR73 halogen moisture analyzer at 85°C for 20 minutes.

### Preparation of irbesartan-HCl*1.5 H₂O

160 g of crude irbesartan was suspended in mixture of 1600 ml of water and 160 ml of methanol at room temperature. 400 ml of 3M HCl were added (pH under 1) and the mixture was heated to reflux temperature. The formed solution was filtered and cooled to the room temperature. The pH was adjusted to a value of under pH = 1, if necessary, by addition of 3M HCl. The mixture was then cooled to 0°C and stirred at this temperature for 1 h. The product was filtered, washed with water and dried under reduced pressure at 35°C for 3h to obtain 160 g of crude irbesartan-HCl*1.5 H₂O.

### Recrystallization

160 g of irbesartan-HCl*1.5 H₂O was dissolved in 320 ml of mixture of isopropanol and 5M HCl (10:1; v:v) at reflux temperature. The formed solution was filtered, cooled to room temperature and stirred for 1 h, then cooled to 0°C and stirred for another 0.5 h. The product was filtered, washed with water and dried under reduced pressure at 40 °C for 2 h to obtain 144 g of recrystallized irbesartan-HCl*1.5 H₂O.

### Examples 1-9

Tablets were prepared according to the wet granulation process (A) as follows.

### 1) Preparation of granulates (1a)-(9a)

The respective solid components shown in Table 1 below were homogenized in a high-shear mixer, and ethanol (96 vol.-%) was sprayed over this mixture. The obtained granulate was dried in a drying chamber or a fluid-bed dryer at a specified temperature. The obtained granulate was sieved and the drying loss was determined in the manner described above. The nature and amounts of the solid components and of the ethanol, the drying device, drying temperature and the drying loss of the sieved granulate are shown in Table 1.

**Table 1**

| | **Granulate** | | | | | | | |
|---|---|---|---|---|---|---|---|---|
| | **1a** | **3a** | **4a** | **5a** | **6a** | **7a** | **8a** | **9a** |
| **Components [g]** | | | | | | | | |
| irbesartan-HCl **¹⁾** | 86.11 | 86.11 | 86.11 | 86.11 | 86.11 | 86.11 | 86.11 | 86.11 |
| mannitol | 83.89 | 40.89 | 83.89 | 81.39 | 78.89 | 71.89 | 69.39 | 69.39 |
| LH-21 **²⁾** | 10 | 7.5 | 5 | 5 | 5 | 7 | 7 | 11 |
| Klucel-EF **³⁾** | - | - | 5 | 5 | 5 | 5 | 7.5 | 7.5 |
| Ethanol (96 vol.-%) | 30 | 50 | 42 | 40 | 30 | 30 | 30 | 30 |

| **Drying method/loss** | | | | | | | | |
|---|---|---|---|---|---|---|---|---|
| drying method **⁴⁾** | DC | FBD | FBD | FBD | FBD | FBD | FBD | FBD |
| drying temp. [°C] **⁵⁾** | 35-40 | 47 | 50-55 | 50-55 | 55 | 55 | 55 | 55 |
| drying loss of granulate [wt.-%] | 3.08 | 3.44 | 3.42 | 2.76 | 2-.59 | 2.92 | 3.06 | 3.20 |

| | | | | | | | | |
|---|---|---|---|---|---|---|---|---|
| **¹⁾** as irbesartan-HCl*1.5 H₂O (86.11 g irbesartan-HCl*1.5 H₂O correspond to 75 g free irbesartan) **²⁾** LH-21: commercially available low-substituted hydroxypropylcellulose **³⁾** Klucel-EF: commercially available low-substituted hydroxypropylcellulose **⁴⁾** DC: drying chamber FBD: fluid-bed dryer **⁵⁾** inlet air temperature | | | | | | | | |

### 2) Preparation of compression mixtures (1b)-(9b)

The components shown in Table 2 below were added to the respective granulates (la)-(9a) obtained above and mixed in a high-shear mixer to obtain a compression mixture. The drying loss of the compression mixtures was determined in the manner mentioned above. The amounts of the added components as well as the drying loss of the compression mixtures are shown in Table 2.

**Table 2**

| | **Compression Mixture** | | | | | | | |
|---|---|---|---|---|---|---|---|---|
| | **1b** | **3b** | **4b** | **5b** | **6b** | **7b** | **8b** | **9b** |
| Granulate | 1a | 3a | 4a | 5a | 6a | 7a | 8a | 9a |

| **Components [g]** | | | | | | | | |
|---|---|---|---|---|---|---|---|---|
| LH-11 **¹⁾** | 10 | 7.5 | 5 | 7.5 | 10 | 15 | 15 | 11 |
| macrogol 6000 | 6 | 5 | 6 | 6 | 6 | 6 | 6 | 6 |
| talc | - | - | 5 | 5 | 5 | 5 | 5 | 5 |
| hydrogenated castor oil | 4 | 3 | 4 | 4 | 4 | 4 | 4 | 4 |
| drying loss of granulate [wt.-%] | 3.16 | 3.82 | 3.15 | 2.66 | 3.05 | 3.12 | 3.07 | 3.07 |

| | | | | | | | | |
|---|---|---|---|---|---|---|---|---|
| **¹⁾** LH-11: commercially available low-substituted hydroxypropylcellulose | | | | | | | | |

### 3) Preparation of compressed tablet cores

The compression mixtures (1b)-(9b) were compressed into oval tablets cores (1c)-(9c) with a theoretical weight of 150 or 200 mg. Also, the compression mixtures (6b)-(9b) were compressed into oval tablet cores (6d)-(9d) with a theoretical weight of 800 mg. The hardness of the compressed tablet cores and the disintegration time (in seconds or minutes) thereof in purified water at 37°C was measured according to Ph. Eur.. The results are shown in Tables 3A and 3B.

**Table 3A**

| | **Compressed Tablet Cores** | | | | | | | |
|---|---|---|---|---|---|---|---|---|
| | **1c** | **3c** | **4c** | **5c** | **6c** | **7c** | **8c** | **9c** |
| Compr. Mixture | 1b | 3b | 4b | 5b | 6b | 7b | 8b | 9b |
| Weight [mg] | 200 | 150 | 200 | 200 | 200 | 200 | 200 | 200 |
| Hardness [N] | 46-82 | 50-66 | 70-98 | 85-101 | 85-105 | 91-104 | 91-107 | 99-113 |
| Disintegration time | 35 sec. | 1 min. | 4-4.5 min. | 3.5 min. | 2 min. | 2-2.5 min. | 2-2.5 min. | 3-3.5 min. |

**Table 3B**

| | **6d** | **7d** | **8d** | **9d** |
|---|---|---|---|---|
| Compression Mixture | 6b | 7b | 8b | 9b |
| Weight [mg] | 800 | 800 | 800 | 800 |
| Hardness [N] | 159-181 | 175-199 | 180-196 | 177-207 |
| Disintegration time | 2 min. | 2.5 min. | 2.5-3 min. | 3-3.5 min. |

In addition, the compressed tablet cores (4c)-(9C) and (6d)-(9d) were coated in an automatic coating pan with water-based film coating suspension of a ready-to-make mixture, commercially available as Opadry II HP. The theoretical weight of the coated tablets containing the cores (4c)-(9C) was 206 mg, and that of the coated tablets containing the cores (6d)-(9d) was 824 mg. The resulting coating thickness is appropriate to hide the unpleasant taste of irbesartan experienced when this is dissolved into the saliva.

### Example 10

Tablets were prepared according to the direct compression process (b) as follows.

86.11 g irbesartan-HCl, 40.89 g mannitol and 8 g croscarmellose sodium were homogenized in high-shear mixer. 1.50 g magnesium stearate, 1.5 g anhydrous silicon dioxide and 12 g of talc were added thereto, thus obtaining a compression mixture. The drying loss thereof, determined in the manner described above, was 3.1 wt.-%.

The compression mixture was compressed into oval tablets with theoretical weight of 150 mg. The hardness of cores was 45-60 N and disintegration time in purified water at 37°C was 20 seconds.

A comparison of the dissolution properties of the tablet cores (4c)-(9c) of the present Examples 4-9 containing 75 mg free irbesartan (as irbesartan-HCl*1.5 H₂O, 86.11 mg) with a pharmaceutical dosage form currently on the market (Aprovel 75 mg tablets) is shown in Fig. 1. As can be seen, the pharmaceutical formulations of the invention show higher dissolution rates after an induction period of about 15 minutes, thus showing an improved quick-release behavior of the drug into the living body.

Dissolution rates were measured on a Dissolution tester ErwekaDT80 with an Agilent Diode Array Spectrophotometer 8453.

### Examples 11-16

Tablets were prepared according to the wet granulation process (A) with addition of hydrochlorothiazide as follows.

### 1) Preparation of granulates (11a)-(18a)

The respective solid components shown in Table 4 below were homogenized in a high-shear mixer, and ethanol (96 vol.-%) was sprayed over this mixture. The obtained granulate was dried in a drying chamber or a fluid-bed dryer. The nature and amounts of the solid components are shown in Table 4.

**Table 4**

| | **Granulate** | | | | | | |
|---|---|---|---|---|---|---|---|
| | **11a** | **13a** | **14a** | **15a** | **16a** | **17a** | **18a** |
| **Components [g]** | | | | | | | |
| Irbesartan-HCl **¹⁾** | 86.11 | 86.11 | 86.11 | 86.11 | 86.11 | 86.11 | 86.11 |
| Mannitol | 71.39 | 71.39 | 68.89 | 66.39 | 59.39 | 56.89 | 56.89 |
| LH-21 **²⁾** | 10 | 5 | 5 | 5 | 7 | 7 | 11 |
| Ethanol (96 vol.%) **³⁾** | q.s. | q.s. | q.s. | q.s. | q.s. | q.s. | q.s. |

| | | | | | | | |
|---|---|---|---|---|---|---|---|
| **¹⁾** as irbesartan-HCl*1.5 H₂O (86.11 g irbesartan-HCl*1.5 H₂O correspond to 75 g free irbesartan) **²⁾** LH-21: commercially available low-substituted hydroxypropylcellulose **³⁾** q.s.: -quantum satis | | | | | | | |

### 2) Preparation of compression mixtures (11b)-(18b)

The components shown in Table 5 below were added to the respective granulates (11a)-(18a) obtained above and mixed in a high-shear mixer to obtain a compression mixture. The amounts of the added components are also shown in Table 5.

### 3) Preparation of compressed tablet cores

The compression mixtures (11b)-(18b) were compressed into tablet cores with a theoretical weight of 200 mg.

In addition, the compressed tablet cores were coated in an automatic coating pan with water-based film coating suspension of a ready-to-make mixture, commercially available as Opadry II HP. The theoretical weight of the coated tablets cores was 206 mg.

**Table 5**

| | **Compression Mixture** | | | | | | |
|---|---|---|---|---|---|---|---|
| | **11b** | **13b** | **14b** | **15b** | **16b** | **17b** | **18b** |
| Granulate | 11a | 13a | 14a | 15a | 16a | 17a | 18a |

| **Components [g]** | | | | | | | |
|---|---|---|---|---|---|---|---|
| Hydrochlorothiazide | 12.5 | 12.5 | 12.5 | 12.5 | 12.5 | 12.5 | 12.5 |
| LH-11 **¹⁾** | 10 | 5 | 7.5 | 10 | 15 | 15 | 11 |
| Macrogol 6000 | 6 | 6 | 6 | 6 | 6 | 6 | 6 |
| Talc | - | 5 | 5 | 5 | 5 | 5 | 5 |
| Hydrogenated castor oil | 4 | 4 | 4 | 4 | 4 | 4 | 4 |

| | | | | | | | |
|---|---|---|---|---|---|---|---|
| **¹⁾** LH-11: commercially available low-substituted hydroxypropylcellulose | | | | | | | |

## Claims

1. A solid pharmaceutical formulation comprising, as an active ingredient, irbesartan hydrochloride and optionally hydrochlorothiazide, mannitol, low-substituted hydroxypropylcellulose and at least one lubricant selected from macrogol, talc and hydrogenated castor oil.

2. The formulation of claim 1, wherein the irbesartan hydrochloride has an average particle size of less than 250 µm.

3. The formulation of any of the preceding claims, wherein the irbesartan hydrochloride has a maximum diameter of 2000 µm.

4. The formulation of any of the preceding claims, wherein the irbesartan hydrochloride is present in an amount of 20-77 wt.-%, based on the total weight of the formulation.

5. The formulation of any of the preceding claims, wherein the low-substituted hydroxypropylcellulose is present in an amount of 1-30 wt.-%, based on the total weight of the formulation.

6. A solid pharmaceutical formulation according to any of the preceding claims, **characterized in that** it is surfactant-free.

7. A process for the manufacture of a formulation as defined in any of claims 1-6, which is a wet granulation process
(A) comprising the following steps:
(i) providing irbesartan hydrochloride having a maximum particle diameter of not more than 2000 µm,
(ii) preparing a compression mixture by using a wetting liquid comprising alcohol, optionally water, a mixture thereof or an aqueous, alcoholic or aqueous/alcoholic granulating liquid by
• (a1) granulating a mixture of one or more excipient(s) and the wetting liquid to obtain granulate, and (a2) adding the irbesartan hydrochloride and optionally further excipient(s) to the granulate to obtain a compression mixture;
• (b1) granulating a mixture of one or more excipient(s), the irbesartan hydrochloride and the wetting liquid to obtain granulate, and (b2) optionally adding further excipient(s) to the granulate to obtain a compression mixture; or
• (c1) granulating a mixture of one or more excipient(s), a portion of the irbesartan hydrochloride and the wetting liquid to obtain granulate, and (c2) adding the remaining irbesartan hydrochloride and optionally further excipient(s) to the granulate to obtain a compression mixture; and
(iii)compressing the compression mixture to the desired form.

8. The process of claim 7, wherein hydrochlorothiazide is added as a further active ingredient in step (ii) in any of the steps (a1) and/or (a2), (b1) and/or (b2), and (c1) and/or (c2).

9. A process for the manufacture of a formulation as defined in any of claims 1-6, which is a direct granulation process (B) comprising the following steps:
(i) providing irbesartan hydrochloride having a maximum particle diameter of not more than 2000 µm,
(ii') mixing the irbesartan hydrochloride and one or more excipient(s) to give compression mixture, and
(iii) compressing the compression mixture to the desired form.

10. The process of claim 9, wherein hydrochlorothiazide is added as a further active ingredient in step (ii)'.

11. The process of any of claims 7-10, which further comprises the step of
(iv) applying a coating to the compressed product obtained in step (iii).

## Patentansprüche

1. Feste pharmazeutische Formulierung, umfassend Irbesartanhydrochlorid und ggf. Hydrochlorothiazid als Wirkstoff, Mannitol, niedrig-substituierte Hydroxypropoylcellulose und wenigstens ein Gleitmittel ausgewählt aus Macrogol, Talk und hydriertem Rizinusöl.

2. Formulierung gemäß Anspruch 1, wobei das Irbesartanhydrochlorid eine mittlere Teilchengröße von weniger als 250 µm aufweist.

3. Formulierung gemäß einem der vorstehenden Ansprüche, wobei das Irbesartanhydrochlorid einen maximalen Durchmesser von 2.000 µm aufweist.

4. Formulierung gemäß einem der vorstehenden Ansprüche, wobei das Irbesartanhydrochlorid in einer Menge von 20 bis 77 Gew.%, bezogen auf das Gesamtgewicht der Formulierung, vorliegt.

5. Formulierung gemäß einem der vorstehenden Ansprüche, wobei die niedrig-substituierte Hydroxypropylcellulose in einer Menge von 1 bis 30 Gew.%, bezogen auf das Gesamtgewicht der Formulierung, vorliegt.

6. Feste pharmazeutische Formulierung gemäß einem der vorstehenden Ansprüche, **dadurch gekennzeichnet, dass** sie Tensid-frei ist.

7. Verfahren zur Herstellung einer Formulierung wie in einem der Ansprüche 1 bis 6 definiert, das ein Nassgranulierungsverfahren (A) ist, das die folgenden Schritte umfasst:
(i) Bereitstellen von Irbesartanhydrochlorid mit einem maximalen Teilchendurchmesser von nicht mehr als 2.000 µm,
(ii) Herstellen einer Pressmischung unter Verwendung einer Befeuchtungsflüssigkeit, umfassend Alkohol, ggf. Wasser, eine Mischung davon oder eine wässrige, alkoholische oder wässrige/alkoholische Granulierungsflüssigkeit durch
• (a1) Granulieren einer Mischung von einem oder mehreren Exzipienten und der Befeuchtungsflüssigkeit, um ein Granulat zu erhalten, und (a2) Zusetzen des Irbesartanhydrochlorids und ggf. weiterer Exzipienten zu dem Granulat, um eine Pressmischung zu erhalten;
• (b1) Granulieren einer Mischung von einem oder mehreren Exzipienten, des Irbesartanhydrochlorids und der Befeuchtungsflüssigkeit, um ein Granulat zu erhalten, und (b2) ggf. Zusetzen weiterer Exzipienten zu dem Granulat, um eine Pressmischung zu erhalten; oder
• (c1) Granulieren einer Mischung von einem oder mehreren Exzipienten, eines Teils des Irbesartanhydrochlorids und der Befeuchtungsflüssigkeit, um ein Granulat zu erhalten, und (c2) Zusetzen des restlichen Irbesartanhydrochlorids und ggf. weiterer Exzipienten zu dem Granulat, um eine Pressmischung zu erhalten; und
(iii) Pressen der Pressmischung in die gewünschte Form.

8. Verfahren gemäß Anspruch 7, wobei Hydrochlorothiazid im Schritt (ii) in einem der Schritte (a1) und/oder (a2), (b1) und/oder (b2) und (c1) und/oder (c2) als weiterer Wirkstoff zugesetzt wird.

9. Verfahren zur Herstellung einer Formulierung wie in einem der Ansprüche 1 bis 6 definiert, das ein Direktgranulierverfahren (B) ist, das die folgenden Schritte umfasst:
(i) Bereitstellen von Irbesartanhydrochlorid mit einem maximalen Teilchendurchmesser von nicht mehr als 2.000 µm,
(ii') Mischen des Irbesartanhydrochlorids und eines oder mehrerer Exzipienten, um die Pressmischung zu ergeben, und
(iii) Pressen der Pressmischung in die gewünschte Form.

10. Verfahren gemäß Anspruch 9, wobei Hydrochlorothiazid in Schritt (ii') als weiterer Wirkstoff zugesetzt wird.

11. Verfahren gemäß einem der Ansprüche 7 bis 10, das ferner den Schritt
(iv) Aufbringen einer Beschichtung auf das in Schritt (iii) erhaltene gepresste Produkt
umfasst.

## Revendications

1. Formulation pharmaceutique solide comprenant, comme un ingrédient actif, du chlorhydrate d'irbésartan et facultativement de l'hydrochlorothiazide, du mannitol, une hydroxypropylcellulose faiblement substituée et au moins un lubrifiant choisi parmi un macrogol, le talc et une huile de ricin hydrogénée.

2. Formulation selon la revendication 1, dans laquelle le chlorhydrate d'irbésartan a une taille moyenne de particules de moins de 250 µm.

3. Formulation selon l'une quelconque des revendications précédentes, dans laquelle le chlorhydrate d'irbésartan a un diamètre maximum de 2 000 µm.

4. Formulation selon l'une quelconque des revendications précédentes, dans laquelle le chlorhydrate d'irbésartan est présent dans une quantité de 20 à 77% en poids, sur la base du poids total de la formulation.

5. Formulation selon l'une quelconque des revendications précédentes, dans laquelle l'hydroxypropylcellulose faiblement substituée est présente dans une quantité de 1 à 30% en poids, sur la base du poids total de la formulation.

6. Formulation pharmaceutique solide selon l'une quelconque des revendications précédentes, **caractérisée en ce qu'**elle est dépourvue d'agent tensioactif.

7. Procédé de fabrication d'une formulation selon l'une quelconque des revendications 1 à 6, qui est un procédé de granulation par voie humide (A) comprenant les étapes suivantes :
(i) prévision d'un chlorhydrate d'irbésartan ayant un diamètre maximum de particules de pas plus de 2 000 µm,
(ii) préparation d'un mélange pour compression en utilisant un liquide de mouillage comprenant un alcool, facultativement de l'eau, un mélange de ceux-ci ou un liquide de granulation aqueux, alcoolique ou aqueux/alcoolique
• (a1) en granulant un mélange d'un ou plusieurs excipient(s) et du liquide de mouillage pour obtenir un granulat, et (a2) en ajoutant le chlorhydrate d'irbésartan et facultativement d'autre(s) excipient(s) au granulat pour obtenir un mélange pour compression ;
• (b1) en granulant un mélange d'un ou plusieurs excipient(s), du chlorhydrate d'irbésartan et du liquide de mouillage pour obtenir un granulat, et (b2) en ajoutant facultativement d'autre(s) excipient(s) au granulat pour obtenir un mélange pour compression ; ou
• (c1) en granulant un mélange d'un ou plusieurs excipient(s), d'une partie du chlorhydrate d'irbésartan et du liquide de mouillage pour obtenir un granulat, et (c2) en ajoutant le reste du chlorhydrate d'irbésartan et facultativement d'autre(s) excipient(s) au granulat pour obtenir un mélange pour compression ; et
(iii) compression du mélange pour compression à la forme désirée.

8. Procédé selon la revendication 7, dans lequel l'hydrochlorothiazide est ajouté comme un autre ingrédient actif à l'étape (ii) dans l'une quelconque des étapes (a1) et/ou (a2), (b1) et/ou (b2), et (c1) et/ou (c2).

9. Procédé de fabrication d'une formulation selon l'une quelconque des revendications 1 à 6, qui est un procédé de granulation direct (B) comprenant les étapes suivantes :
(i) prévision d'un chlorhydrate d'irbésartan ayant un diamètre maximum de particules de pas plus de 2 000 µm,
(ii') mélangeage du chlorhydrate d'irbésartan et d'un ou plusieurs excipient(s) pour donner un mélange pour compression, et
(iii) compression du mélange pour compression à la forme désirée.

10. Procédé selon la revendication 9, dans lequel l'hydrochlorothiazide est ajouté comme un autre ingrédient actif à l'étape (ii').

11. Procédé selon l'une quelconque des revendications 7 à 10, qui comprend en outre l'étape de
(iv) application d'un enrobage au produit comprimé obtenu à l'étape (iii).
